(19) Europäisches Patentamt
European Patent Office
Office européen des brevets

(11) Numéro de publication : **0 016 702**
**B1**

(12) **FASCICULE DE BREVET EUROPÉEN**

(45) Date de publication du fascicule du brevet : **20.02.85**

(21) Numéro de dépôt : **80400353.1**

(22) Date de dépôt : **17.03.80**

(51) Int. Cl.⁴ : **A 61 K 9/18**, A 61 K 35/30, A 61 K 31/73

(54) **Médicament, notamment pour le traitement du choléra, et compositions pharmaceutiques le contenant.**

(30) Priorité : **16.03.79 FR 7906732**
**16.03.79 DE 2910509**

(43) Date de publication de la demande :
**01.10.80 Bulletin 80/20**

(45) Mention de la délivrance du brevet :
**20.02.85 Bulletin 85/08**

(84) Etats contractants désignés :
**BE CH DE FR GB IT NL**

(56) Documents cités :
**FR-A- 2 320 760**
**GB-A- 2 004 186**
**CHEMICAL ABSTRACTS, vol. 90, no. 11, 12 mars 1979, page 136, ref. 81812z, Columbus, Ohio, US, J.L. TAYOT et al.: "Biospecific chromatography on new derivatives of porous silica beads. Coupling of ganglioside Gm1 of anticholeragen antibody for purification of cholera toxin"**
**CHEMICAL ABSTRACTS, vol. 83, no. 17, 27 octobre 1975, page 117, ref. 142735f, Columbus, Ohio, US, SAM T. DONTA et al.: "Inhibition of the steroidogenic effects of cholera and heat-labile Escherichia coli enterotoxins by Gm1 ganglioside. Evidence for a similar receptor site for the two toxins"**
**CHEMICAL ABSTRACTS, vol. 83, no. 21, 24 novembre 1975, page 130, ref. 173587p, Columbus, Ohio, US, J. HOLMGREN et al.: "Interaction of cholera toxin and membrane Gm1 ganglioside of small intestine"**
**Le dossier contient des informations techniques présentées postérieurement au dépôt de la demande et ne figurant pas dans le présent fascicule.**

(73) Titulaire : **INSTITUT MERIEUX Société anonyme dite: 17, rue Bourgelat F-69002 LYON (FR)**

(72) Inventeur : **Mynard, Marie-Claude**
**246, rue du Commandant Charcot**
**F-69110 Sainte-Foy-lès-Lyon (FR)**
Inventeur : **Tayot, Jean-Louis**
**1, rue des Greffières**
**F-69890 La Tour de Salvagny (FR)**

(74) Mandataire : **Nony, Michel et al**
**Cabinet Nony 29, rue Cambacérès**
**F-75008 Paris (FR)**

**EP 0 016 702 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un nouveau médicament et les compositions pharmaceutiques le contenant.

L'invention a plus précisément pour objet un nouveau médicament permettant de traiter, à titre prophylactique ou à titre curatif, les diarrhées aiguës et notamment le choléra.

Le médicament de l'invention est caractérisé par le fait qu'il est constitué d'un support sur lequel est fixé un ganglioside ou un dérivé de ganglioside doué d'affinité pour les toxines produites par les bactéries responsables des diarrhées et en particulier pour la toxine cholérique, ledit support étant compatible avec une administration par voie orale.

Les supports utilisables sont tous ceux qui sont compatibles avec des utilisations par voie orale, qui possèdent une affinité forte, de préférence irréversible pour les gangliosides ou qui peuvent être liés chimiquement avec les gangliosides. Ils doivent présenter de préférence une surface spécifique élevée (utilisation de supports poreux). Ils doivent en outre permettre un contact satisfaisant entre le ganglioside et les toxines à neutraliser.

Les supports sont choisis par exemple dans le groupe constitué par : le charbon activé, le latex, des polysaccharides tels que la cellulose modifiée ou non, et les supports minéraux poreux tels que la silice, l'alumine, l'oxyde de titane, ou leurs dérivés naturels ou synthétiques tels que des verres, des silicates, le kaolin, etc..., éventuellement revêtus de polysaccharides.

De préférence, le support est le charbon activé de qualité pharmaceutique, et en particulier le charbon commercialisé sous la dénomination ACTI-CARBONE WL 3S.

D'une manière générale, tous les charbons de qualité pharmaceutique peuvent convenir.

On sait que parmi les divers gangliosides, le ganglioside $G_{M1}$ et certains de ses dérivés sont doués d'une affinité de type biochimique forte et spécifique pour les toxines cholériques et les toxines structurellement apparentées. Cette affinité est également observée avec certains dérivés du ganglioside $G_{M1}$. C'est en particulier le cas du lysoganglioside $G_{M1}$. Le lysoganglioside $G_{M1}$ est obtenu par hydrolyse alcaline du ganglioside $G_{M1}$, selon le procédé décrit par HOLMGREN, MÄNSSON et SVENNERHOLM, Medical Biology (1974), 52, 229-233. Cette hydrolyse alcaline a pour but de transformer les deux fonctions N-acétyle et la fonction N-acyle du ganglioside $G_{M1}$ en fonction amine $NH_2$.

D'autres dérivés de gangliosides ont également une affinité pour la toxine cholérique.

Dans la présente demande, on appellera par convention « dérivé du ganglioside $G_{M1}$ » ou « $G_{M1}$ », tout dérivé de ganglioside qui a une affinité pour la toxine cholérique, et notamment les produits résultant d'une hydrolyse partielle ou totale des groupements N-acyle ou N-acétyle en groupements $-NH_2$, du ganglioside $G_{M1}$, mais aussi les mono- ou a-sialo gangliosides résultant du traitement acide des gangliosides ou de leurs dérivés (notamment de leurs dérivés résultant de l'hydrolyse totale ou partielle des groupements N-acyle ou N-acétyle).

Les produits de désacylation partielle des gangliosides présentent des groupements amine libre pouvant être mis en évidence par une réaction positive dans le test à la ninhydrine, sont mobiles en chromatographie sur couche mince de gel de silice dans le système chloroforme-méthanol-eau (60 : 32 : 7), et présentent les propriétés d'affinité spécifique des gangliosides dont ils dérivent. Ils conservent ces propriétés d'affinité même s'ils sont couplés à des supports solides, par l'intermédiaire des groupements amine apparus lors de la désacylation partielle.

Ils sont préparés par traitement des gangliosides avec une solution aqueuse basique à une température comprise entre 0 et 120 °C, ladite température étant d'autant plus basse que le milieu est plus basique. Pour une solution finale d'alcanilité comparable à la potasse ou la soude normale, il importe de ne pas dépasser 100 °C. Pour une alcalinité comparable à la potasse ou la soude décinormale, il importe de ne pas dépasser 120 °C.

Le temps de réaction doit être suffisant pour qu'apparaissent des groupements amine libre, mais il faut en outre arrêter la réaction avant désacylation totale. Le temps de réaction peut être déterminé facilement dans chaque cas par une expérimentation simple, en utilisant par exemple le test à la ninhydrine, en recherchant une coloration intermédiaire d'intensité inférieure à celle obtenue après désacylation complète. Généralement, le temps de réaction varie de 30 minutes à 24 heures.

Le solvant de réaction est soit l'eau, soit un mélange d'eau et de solvants organiques. Parmi les solvants organiques on citera des alcools tels que le butanol.

Parmi les bases utilisables, on citera celles qui sont capables de donner dans l'eau un pH 9 à 14, et en particulier dissoudre le ganglioside choisi dans de la potasse ou de la soude normale et à incuber cette solution à 60 °C pendant 2 heures.

Quant aux produits de désacylation totale des gangliosides, ils sont préparés par les procédés décrits dans la littérature.

On a en outre découvert que certaines bactéries responsables de diarrhées aiguës, et en particulier certaines souches de Escherichia coli, sont susceptibles de libérer des entérotoxines qui peuvent se lier aux dérivés de gangliosides, en particulier au ganglioside $G_{M1}$.

Généralement, le médicament de l'invention contient de 1 à 20 micromoles environ, et en particulier de 3 à 10 micromoles environ, de gangliosides ou de dérivés de gangliosides par gramme de support.

Le support est généralement sous forme d'un matériau particulaire. Les dimensions des particules du matériau support peuvent varier par exemple de 0,1 à 1 000 micromètres.

L'invention a également pour objet un procédé de préparation du médicament tel que défini précédemment. Ce procédé est caractérisé par le fait que l'on fixe le ganglioside $G_{M1}$ et/ou ses dérivés sur le support. Cette fixation peut être réalisée soit par l'établissement d'une liaison chimique, soit en mettant à profit un phénomène d'affinité quelconque, par exemple un phénomène d'adsorption.

Dans ce dernier cas, il suffit de mettre en contact le support avec une solution de gangliosides ou de dérivés de gangliosides.

Une telle solution de gangliosides ou de dérivés de gangliosides peut être notamment obtenue selon le procédé décrit dans la demande de brevet français n° 2 319 399.

Après un temps de contact suffisant pour coupler le ganglioside au support, on sépare le produit couplé par centrifugation puis on lave le précipité obtenu.

En variante, on peut, par exemple avant ou après centrifugation, transformer le ganglioside en un autre dérivé de ganglioside, notamment par hydrolyse acide qui permet de transformer la majorité des polysialogangliosides en monosialogangliosides.

Généralement, la fixation du ganglioside ou de ses dérivés sur le support est suffisamment résistante. Elle peut éventuellement être complétée par une réaction chimique de couplage.

En ce qui concerne les particules de charbon actif, la simple fixation par adsorption est suffisante. Le ganglioside $G_{M1}$ ou ses dérivés est facilement adsorbé.

Pour fixer le ganglioside $G_{M1}$ sur des particules de latex, on opère à pH alcalin, alors que ce pH alcalin n'est pas nécessaire pour coupler le lysoganglioside $G_{M1}$. Lorsque le latex est porteur de fonctions $NH_2$, il est utile de le prétraiter avec un dérivé carbonylé, un tel prétraitement favorisant la fixation du ganglioside ou de ses dérivés.

Le dérivé de ganglioside peut être fixé par exemple sur des particules de polysaccharides. L'expression « polysaccharide » englobe, dans la présente demande, les polysaccharides modifiés. Ces polysaccharides sont notamment du dextran, des celluloses, de l'amidon, de l'agarose, etc... ou encore des polysaccharides modifiés, notamment des dialkylaminoalkyl- ou di (hydroxyalkyl) aminoalkyl-polysaccharides tels que par exemple le diéthylaminoéthyl dextran, la diétylaminoéthyl cellulose, etc... On fixe le dérivé de ganglioside sur les polysaccharides à l'aide d'agents de couplage tels que des diépoxydes, des dicarbonylés, des épihalohydrines ou le bromure de cyanogène, selon les méthodes connues.

Les polysaccharides peuvent être également utilisés, non pas sous forme de particules, mais sous forme de revêtement sur des particules minérales, notamment des oxydes minéraux tels que ceux mentionnés ci-dessus.

Les particules minérales poreuses revêtues de polysaccharides ou de polysaccharides modifiés peuvent être notamment celles décrites dans la demande de brevet français n° 2 319 399, ou celles décrites dans la demande de brevet français n° 2 403 098.

Les particules minérales revêtues de polysaccharides, conformément à la demande de brevet français n° 7 623 176 sont constituées d'un support minéral poreux, tel qu'un oxyde minéral poreux, revêtu directement sur sa surface par un polymère polysaccharidique aminé.

Le support minéral poreux peut être de la silice, de l'alumine, de la magnésie, un oxyde de titane, ou leurs dérivés synthétiques ou naturels tels que verres, borosilicates, silicates, kaolin, etc...

Le polymère polysaccharidique aminé est fixé sur le support minéral poreux par encollage.

La surface interne du support minéral poreux est par exemple inférieure ou égale à 100 $m^2/g$ et si possible comprise entre 5 et 80 $m^2/g$. Le diamètre poreux moyen est de préférence supérieur ou égal à 25 nm et si possible compris entre 50 et 1 000 nm. Pour des surfaces plus grandes ou des diamètres poreux faibles la surface interne du support devient inaccessible au polymère polysaccharidique. Le support poreux minéral est par exemple de la silice ou de l'alumine et de préférence un support de silice poreuse à caractère anionique obtenue selon les procédés décrits dans les brevets français n° 1 473 239, n° 1 473 240, n° 1 475 929, n° 1 482 867, telles que les silices poreuses commercialisées par RHONE-POULENC CHIMIE FINE sous les dénominations SPHEROSIL XOB 030, XOB 015, XOB 005 et XOC 005.

Le polymère polysaccharidique aminé qui sert à imprégner et à recouvrir la surface interne du support poreux minéral doit avoir un caractère cationique prononcé et avoir de bonnes propriétés d'hydrophylie. Il doit avoir un poids moléculaire au moins égal à $10^4$ daltons et si possible être compris entre $10^5$ et $10^6$. Sa formule peut être quelconque et il peut en particulier être un dérivé aminé du dextran, de l'amidon, de la cellulose, de l'agarose ou d'un polymère naturel ou synthétique de tous les oses connus.

Les fonctions amines du polymère polysaccharidique peuvent être primaires, secondaires ou tertiaires ou éventuellement quaternaires.

Le polymère polysaccharidique aminé peut notamment correspondre à la formule

$$R—(CH_2)_n—NR_1(R_2)$$

dans laquelle :

R représente un reste de polysaccharide tel que par exemple un reste de dextran, d'amidon, de cellulose ou d'agarose,

n est un nombre entier de 1 à 10 et de préférence de 2 à 5,

$R_1$ et $R_2$ identiques ou différents représentent un radical alkyle ou hydroxyalkyle inférieur, par exemple les radicaux suivants :

$—CH_3$, $—CH_2—CH_3$, $—CH_2OH$, $—CH_2—CH_2OH$ ou $—CH_2—CHOH—CH_3$, ces polymères pouvant être quaternarisés à l'aide d'un agent quaternarisant classique tel que les halogénures d'alkyle ou d'hydroxyalkyle, le sulfate de diméthyle, etc...

Parmi les polymères de ce type, on peut en particulier citer les composés connus sous les dénominations DEAE DEXTRAN (diéthylamino éthyl dextran) de poids moléculaire 500 000 et QAE DEXTRAN (diéthylamino éthyl dextran quaternisé) vendus par la firme PHARMACIA et le composé connu sous la dénomination DEAE amidon (diéthylamino éthyl amidon) ainsi que les amidons cationiques tels que ceux vendus sous la dénomination commerciale CATO par la Société ROQUETTE NATIONAL.

Le polymère polysaccharidique aminé peut aussi être réticulé à l'aide d'un agent réticulant par exemple un composé dicarbonylé, un diépoxyde tel que le 1-4 butanedioldiglycidyléther, ou l'épichlorhydrine ou l'épibromhydrine.

On fixe les dérivés de gangliosides sur les supports poreux ainsi revêtus, par exemple à l'aide des agents de couplage déjà mentionnés ci-dessus.

Les particules minérales poreuses revêtues de polysaccharides modifiés ou non, selon la demande de brevet français n° 2 403 098, sont constituées par un support minéral poreux revêtu par un polymère polysaccharidique, ou par un polymère polysaccharidique modifié, par exemple un polysaccharide amine tel que mentionné ci-dessus, ledit revêtement polysaccharidique étant si nécessaire stabilisé par réticulation et sur ledit revêtement polysaccharidique ou polysaccharidique modifié se trouve greffé un dérivé de ganglioside de formule schématique $R''—NH_2$, R" étant le reste de la molécule du dérivé de ganglioside, la liaison de greffage de ladite molécule répondant à la formule

$$R_3—CH_2—NH—R''$$

R" étant défini comme précédemment et $R_3—CH_2—$ représentant le reste dudit polymère polysaccharidique ou polysaccharidique modifié lorsque celui-ci a été soumis à une réaction de coupure oxydante suivie d'une réduction.

Les supports minéraux poreux sont ceux déjà indiqués précédemment ; le polymère polysaccharidique est notamment la cellulose ; le polymère polysaccharidique modifié est notamment le diéthylaminoéthyldextran, le diéthylaminoéthylamidon, ou la diéthylaminoéthylcellulose ; le revêtement de polymère polysaccharidique modifié ou non est si nécessaire stabilisé par réticulation, l'agent réticulant étant tel que ceux mentionnés ci-dessus.

Le procédé de préparation de ces matériaux est caractérisé par le fait que l'on effectue un revêtement du support minéral poreux par le polymère polysaccharidique ou par le polymère polysaccharidique modifié, que si désiré transforme le revêtement polysaccharidique en revêtement polysaccharidique modifié, que l'on effectue si nécessaire une réticulation pour stabiliser le revêtement, que l'on soumet ledit revêtement polysaccharidique ou polysaccharidique modifié à une réaction de coupure oxydante selon les méthodes connues, que l'on fait réagir le produit d'oxydation ainsi obtenu avec le lysoganglioside $G_{M1}$ ou tout autre dérivé du ganglioside $G_{M1}$ tel que défini ci-dessus, de formule schématique $R''—NH_2$, puis que l'on soumet le dérivé iminé obtenu à l'action d'un agent réducteur capable de réduire la liaison imine en liaison amine.

Le procédé décrit ci-dessus est applicable aux produits de désacylation partielle ou totale des gangliosides car ces produits comportent des groupements $NH_2$.

Comme indiqué ci-dessus le médicament de l'invention possède des propriétés pharmacologiques intéressantes qui permettent de l'utiliser notamment par voie orale, dans le traitement préventif ou curatif du choléra et d'autres diarrhées aiguës.

On a en effet découvert que le médicament de l'invention pouvait être utilisé par voie orale et qu'il permettait de fixer la toxine cholérique ainsi que d'autres toxines bactériennes, éventuellement présentes dans l'intestin.

On sait que le ganglioside $G_{M1}$ et ses dérivés ont une affinité notamment pour la toxine cholérique. Toutefois, une utilisation thérapeutique de cette affinité n'était pas évidente, car il fallait trouver un mode d'administration particulier permettant d'introduire le $G_{M1}$ sous une forme non soluble, dans l'intestin, de façon à ce que le $G_{M1}$ ne soit pas adsorbé, ou absorbé par les cellules de la paroi intestinale, ce qui favoriserait au contraire l'action de la toxine.

Il était donc particulièrement important de trouver une forme d'administration qui permettait de fixer solidement le $G_{M1}$ sur son support insoluble, tout en gardant accessibles les sites actifs nécessaires à la neutralisation de la toxine.

On a trouvé que ces conditions étaient bien réunies dans le médicament de l'invention. Ce médicament est stable et la liaison support-ganglioside résiste à un traitement acide, à l'ébullition en milieu aqueux acide, et à l'action des sels biliaires. Le médicament de l'invention est donc compatible avec une utilisation thérapeutique par voie orale, et manifeste une forte affinité pour les toxines mentionnées ci-dessus.

La présente invention a également pour objet une composition pharmaceutique caractérisée par le fait qu'elle renferme comme principe actif un médicament tel que défini précédemment.

La composition pharmaceutique de l'invention peut être constituée par le médicament défini précédemment, sans excipient. Toutefois, elle contient généralement, en outre, un excipient permettant de la mettre sous une forme galénique compatible avec l'administration par voie orale.

De préférence, la composition pharmaceutique de l'invention contient de 0,1 à 20, et en particulier de 0,1 à 10 micromoles environ de gangliosides ou de dérivés de gangliosides par gramme de composition.

Ces compositions peuvent se présenter notamment sous la forme de paquets ou de sachets de poudre, de cachets, de gélules, de granulés, de comprimés (à croquer, à avaler ou à dissoudre dans de l'eau), de suspensions buvables ou de capsules molles.

La composition pharmaceutique de l'invention peut également contenir en outre au moins un produit actif choisi parmi des antiseptiques intestinaux (benzonaphtol, hexamine, quinoléine), des antibiotiques, des antispasmodiques, etc...

La composition pharmaceutique de l'invention peut également contenir en outre des substances tensioactives, physiologiques ou non, permettant d'assurer une meilleure répartition du médicament dans le tube digestif et donc d'accroître son efficacité.

Ces compositions pharmaceutiques sont préparées selon les techniques usuelles de fabrication des formes galéniques.

Lorsque le support est le charbon activé, la fabrication des formes galéniques est analogue à la fabrication des formes d'administration contenant le charbon activé seul. En particulier, les comprimés sont de préférence obtenus en comprimant un granulé contenant un liant et d'autres excipients, encore à l'état humide puis en séchant les comprimés ainsi préparés.

L'invention a également pour objet le procédé de préparation des compositions pharmaceutiques telles que définies ci-dessus. Ce procédé est principalement caractérisé par le fait que l'on mélange le médicament de l'invention, les autres principes actifs, éventuellement présents, et l'excipient, puis que l'on donne à la composition obtenue la forme désirée, selon les méthodes usuelles.

Comme indiqué ci-dessus, le médicament de l'invention, présenté notamment sous la forme des compositions telles que définies précédemment, peut être utilisé dans le traitement du choléra et d'autres diarrhées aiguës, soit à titre prophylactique, soit à titre curatif.

L'invention a en particulier pour objet un procédé de traitement des diarrhées aiguës et notamment du choléra, ou des diarrhées à Escherichia coli, caractérisé par le fait que l'on administre par voie orale, à un humain ou à un animal souffrant ou risquant de souffrir de telles diarrhées, le médicament défini précédemment.

Outre le choléra, d'autres diarrhées aiguës sont fréquemment produites par des bactéries productrices d'entérotoxines, telles certaines souches d'Escherichia coli, qui sont susceptibles de libérer notamment une toxine thermolabile dont la structure est proche de la toxine cholérique et qui peut se lier au ganglioside $G_{M1}$. Le médicament et le procédé de traitement de l'invention peuvent donc être efficaces, à titre prophylactique ou curatif, notamment contre les diarrhées à Escherichia coli.

Selon un mode d'exécution particulier, le procédé de traitement de l'invention est caractérisé par le fait que l'on administre le médicament à titre prophylactique, en quantité correspondant généralement à l'administration quotidienne de 2 à 50 micromoles de gangliosides ou de dérivés de gangliosides environ. Selon un autre mode d'exécution du procédé de traitement de l'invention, on administre à titre curatif le médicament, en quantité correspondant à une administration quotidienne de 10 à 200 micromoles de gangliosides ou de dérivés de gangliosides environ.

La posologie varie notamment en fonction de l'effet thérapeutique recherché (prophylactique ou curatif), de l'intensité du syndrome diarrhéique, et de l'âge du sujet. La posologie indiquée ci-dessus correspond à celle qui est généralement efficace pour un humain adulte.

Les exemples suivants illustrent l'invention sans toutefois la limiter.

## Exemple 1

### Technique

A partir de cervelles de veau, ou de bœuf, on prépare un extrait aqueux de gangliosides. Ces gangliosides sont purifiés sur colonne de Sphérosil — DEAE — Dextran comme décrit dans la demande de brevet français n° 7 623 176. L'élution est réalisée par un tampon acétate de potassium 0,5 M, dans du méthanol. A cet éluat on ajoute du charbon activé (CECA, qualité WL3S), à raison de 1 gramme par 10 micromoles de gangliosides (ou bien 1 à 20 micromoles). Après centrifugation et lavage, le produit couplé est soumis à une hydrolyse acide à 80 °C pendant 2 heures, avec une solution d'acide formique 0,1 M.

La majorité des polysialogangliosides fixée sur le charbon est transformée en monosialogangliosides.

Après lavage à l'aide d'une solution de bicarbonate de potassium et séchage, on obtient une poudre appelée ci-après charbon-$G_{M1}$.

### Caractéristiques du produit

Aspect :

Poudre de charbon.

Identification

1° Extraction : par ébullition au bain-marie bouillant pendant 1 heure dans un mélange chloroforme/méthanol/eau (60/30/4,5 volume à volume).

2° Par chromatographie en couches minces de l'extrait traité sur colonne de Séphadex G 25, qui montre une tache principale correspondant au $G_{M1}$ purifié.

Dosage

Sur une aliquote de l'extractum précédent, on effectue le dosage de l'acide sialique selon la méthode de SVENNERHOLM, B.B.A. 1957, *24*, 604.

Activité

Exprimée en microgrammes de toxine cholérique pure étalon (OMS), fixée par gramme de charbon-$G_{M1}$.

Pour la détermination de cette activité, un test in vitro ou un test in vivo peut être utilisé.

Test in vitro : neutralisation d'un taux exact connu de toxine cholérique par le charbon-$G_{M1}$, puis titrage de la toxine résiduelle par immunodiffusion (technique de MANCINI) et/ou par hémagglutination (agglutination de globules rouges de mouton recouverts de $G_{M1}$).

Test in vivo : neutralisation d'un taux exact connu de toxine cholérique par le charbon-$G_{M1}$ et inoculation de la suspension dans l'anse iléale de lapin).

Stabilité

Conservation en flacon fermé, à température ambiante. Le produit est stable. Le produit n'est pas affecté par une conservation à 50 °C. La liaison charbon-$G_{M1}$ n'est pas affectée par des conditions de pH acide ou alcalin, ni par l'action des sels biliaires.

## Exemple 2

Préparation de granulés vermiculés :

Composition :

| | |
|---|---|
| Poudre de charbon-$G_{M1}$ | 100 g |
| Sucre glace | 740 g |
| Sirop simple (environ) | 250 g |

On mélange sucre glace et charbon dans un mortier ; on ajoute peu à peu le sirop pour obtenir une pâte qui donne des granulés vermiculés par passage au travers des mailles d'un tamis.

## Exemple 3

Préparation de granulés :

Composition :

| | |
|---|---|
| Poudre de charbon-$G_{M1}$ | 50 g |
| Saccharose | 44 g |
| Gomme arabique | 6 g |

On mouille le charbon maintenu sous agitation à l'aide d'un sirop amenant 70 g d'eau purifiée, 44 g de saccharose et 6 g de gomme arabique. On granule. On sèche en étuve à 50 °C.

La gomme arabique intervient comme liant. On peut également utiliser à sa place un des nombreux amidons prégélatinisés du commerce, modifiés ou non.

## Exemple 4

Préparation de comprimés :

Composition :

| | |
|---|---|
| Poudre de charbon-$G_{M1}$ | 40 g |
| Saccharose | 54 g |
| Amidon de maïs prégélatinisé | 6 g |

On introduit le charbon-$G_{M1}$ dans un mortier. On mouille par un sirop apportant 60ml d'eau, 54 g de saccharose et 6 g d'amidon prégélatinisé. On granule. On sèche partiellement le granulé à 50 °C en étuve ventilée pendant environ 1 heure. On comprime le granulé encore très légèrement humide. On sèche à 50 °C pendant 18 heures les comprimés obtenus.

On prépare ainsi des granulés très durs, peu friables, mais qui se délitent en moins de 15 minutes selon le protocole d'essais de la Pharmacopée Française 9ème édition.

Exemple 5

Administration du médicament :

On utilise une composition à base de charbon-$G_{M1}$ contenant 5 micromoles de $G_{M1}$ par gramme.

A titre prophylactique, on administre 2 g par jour de cette composition.

A titre curatif, en période de diarrhées aiguës, on administre 1 g toutes les 1 à 2 heures, puis 1 g toutes les 4 à 6 heures.

Exemple 6

Préparation de sachets ou de gélules de Cellulose-$G_{M1}$

Composition :

| | |
|---|---|
| Cellulose-$G_{M1}$ | 70 g |
| Sucre glace | 29 g |
| Stéarate de magnésium | 1 g |

Mélanger à sec puis répartir en sachets ou en gélules.

La cellulose-$G_{M1}$ contient une micromole par gramme, capable de neutraliser jusqu'à 6 mg de toxine cholérique par gramme.

Exemple 7

Préparation de comprimés de Cellulose-$G_{M1}$

Comprimer la poudre de Cellulose-$G_{M1}$ pour préparer des comprimés plats d'un diamètre de 30 mm. Les broyer pour obtenir un grain passant au travers d'un tamis de 300 µm. Ajouter 1 % de Stéarate de magnésium. Comprimer pour obtenir des comprimés de 10 mm de diamètre, bombés.

La Cellulose-$G_{M1}$ est la même qu'à l'exemple 6.

Exemple 8

Préparation de comprimés de Cellulose-$G_{M1}$

Mouiller la poudre de Cellulose-$G_{M1}$ (analogue à celle de l'exemple 6) à l'aide d'une solution aqueuse de PEG 6000 à 20 % dans l'eau purifiée pour apporter, en extrait sec, 2,5 % de PEG à 97,5 % de Cellulose-$G_{M1}$. Passer le mélange humide sur un appareil à granulation. Sécher le granulé obtenu. Le peser et ajouter 1 % de son poids en Stéarate de magnésium. A partir de ce mélange, préparer des comprimés de diamètre 10 mm, bombés.

Exemple 9

Exemples de préparation de dérivés de désacylation partielle de $G_{M1}$

9.1. Activation du glanglioside $G_{M1}$

5 micromoles de $G_{M1}$ sont dissoutes dans 1 ml de soude N et la solution est incubée à 80 °C pendant 2 heures. La solution peut être ensuite diluée et ajustée pour le couplage du ganglioside activé selon l'une quelconque des méthodes décrites plus loin.

Le produit obtenu présente une réaction positive dans le test à la ninhydrine, la coloration observée étant toutefois moins intense que celle observée avec le lysoganglioside $G_{M1}$.

9.2 Activation du ganglioside $G_{M1}$

10 micromoles de $G_{M1}$ sont dissoutes dans 10 ml de soude N/10 et la solution est incubée à 40 °C pendant 15 heures. La solution est alors prête pour le couplage du ganglioside activé selon l'une quelconque des méthodes décrites plus loin. Le test à la ninhydrine et le test de SANGER sont positifs. Dans le cas de couplage sur un support époxy, ce support peut avantageusement être ajouté au début du traitement alcalin. L'activation du ganglioside et sa fixation sur support se font alors dans la même étape au même pH.

9.3. Couplage du ganglioside $G_{M1}$ ainsi activé sur une matrice polysacchraridique par l'intermédiaire d'un agent bifonctionnel à fonctions époxy.

On peut utiliser par exemple la cellulose. Le couplage à l'aide d'un agent diépoxy — par exemple le 1-4 butanedioldiglycidyléther (Aldrich) — peut se faire en incubant pendant une nuit à température ambiante 10 g de cellulose dans un mélange contenant 20 ml de NaOH N, 20 ml de Butanedioldiglycidyléther et 40 mg de $NaBH_4$.

Le lendemain le support est lavé par de l'alcool, de l'eau et enfin de la soude 0,1 N. Après essorage par filtration, le support est mis en contact pendant une nuit avec le ganglioside précédemment activé et en solution dans de la soude 0,1 N. Une dose de 1 à 10 µmole/ml est recommandée. Une température d'incubation de 20 à 40 °C est préférable.

9.4. Couplage du ganglioside $G_{M1}$ ainsi activé sur une matrice polysaccharidique oxydée par le periodate de sodium.

Pour cette méthode, la cellulose ou les supports poreux imprégnés de polysaccharides tels que ceux de la demande de brevet français n° 2 319 399 sont préférables. L'agarose est en effet peu oxydé dans les mêmes conditions.

Cette méthode a déjà été appliquée (voir demande de brevet français n° 2 403 098) pour le couplage des lysogangliosides préparés selon la méthode de TAKETOMI.

Les conditions pour fixer les gangliosides activés par désacylation partielle sont identiques.

10 g de support (par exemple la cellulose) sont additionnés de 100 ml de periodate de sodium 0,02 M pendant 2 heures à température ambiante.

Le support est ensuite lavé dans une solution de carbonate de sodium 0,02 M pH 9 additionnée de NaCl 10 g/l, essoré par filtration et additionné de 15 ml d'une solution à pH 9 de gangliosides activés selon l'un quelconque des exemples 9.1. et 9.2. ci-dessus. Une dose de 1 à 10 µmole/ml de support est recommandée.

Après incubation une nuit à température ambiante, du borohydrure de sodium $NaBH_4$ est ajouté pendant 2 heures à 20 °C (0,2 M en final) pour réduire les liaisons imines formées en liaisons amines extrêmement stables.

9.5. Couplage du ganglioside $G_{M1}$ ainsi activé sur une matrice polysaccharidique par la méthode au bromure de cyanogène.

La méthode est celle décrite par PORATH et al., Nature, 215, 1491 (1967). En pratique, il suffit de mélanger le Sepharose activé au bromure de cyanogène, commercialisé par PHARMACIA (Uppsala-Suède), à pH 11 suivant les instructions de la notice d'emploi. Une dose de 1 à 10 µmole de ganglioside activé par ml de gel est ici encore recommandée. Il est possible d'adapter cette technique à la cellulose ou aux supports minéraux poreux précédemment décrits, sans rien changer aux conditions opératoires.

9.6. Etude de la fixation de la toxine cholérique par le médicament de l'invention.

Le support minéral poreux est de la silice telle que celle commercialisée par RHONE-POULENC sous le nom de SPHEROSIL XOC 005. Sa surface spécifique est d'environ 10 $m^2/g$, son diamètre poreux est d'environ 300 nm ; cette silice est imprégnée d'une couche monomoléculaire de DEAE Dextran et réticulée selon le procédé décrit dans la demande de brevet français n° 2 319 399. En appliquant la méthode décrite aux exemples 9.3, 9.4. ou 9.5. ci-dessus à 1 g de ce support, il est possible de fixer par exemple 5 µmole de ganglioside $G_{M1}$ préalablement activé par désacylation selon les exemples 9.1. ou 9.2. ci-dessus.

Ce support est ensuite lavé par de la soude 0,1 N et monté en colonne. La colonne est équilibrée dans une solution contenant du citrate de sodium 0,05 M à pH 7 additionnée de NaCl 10 g/l. Un filtrat de culture de « Vibrio cholerea INABA 569 B » contenant à l'état brut 25 µg/ml de toxine cholérique est dialysé contre le même tampon. 500 ml de cette solution impure de toxine cholérique sont ensuite filtrés sur la colonne. Toutes les impuretés ne reconnaissant pas le ganglioside $G_{M1}$ traversent la colonne. Seule la toxine cholérique se fixe sur la colonne et peut être récupérée par élution avec de l'acide citrique 0,05 M pH 2,8. Le produit récupéré contient environ 100 % de l'activité biologique présente dans la culture initiale. La toxine cholérique ainsi recueillie (12,5 mg) apparaît identique à celle préparée selon le procédé décrit dans la demande de brevet français n° 2 403 098.

De la même façon, on a réalisé la fixation de ganglioside $G_{M1}$, selon la même méthode, sur d'autres supports réalisés à partir d'agarose ou de cellulose.

**Revendications**

1. Nouveau médicament, caractérisé par le fait qu'il est constitué par un support sur lequel est fixé un ganglioside ou un dérivé de ganglioside doué d'affinité pour les toxines produites par des bactéries responsables des diarrhées, et notamment pour la toxine cholérique, ledit support étant compatible avec une administration par la voie orale.

2. Médicament selon la revendication 1, caractérisé par le fait que le support est choisi dans le groupe constitué par : le charbon activé, le latex, la cellulose modifiée ou non, les supports minéraux poreux tels que la silice, l'alumine, l'oxyde de titane, ou leurs dérivés naturels ou synthétiques tels que des verres, des silicates ou le kaolin.

3. Médicament selon l'une quelconque des revendications précédentes, caractérisé par le fait que le ganglioside ou le dérivé de ganglioside est choisi dans le groupe constitué par : le ganglioside $G_{M1}$, le lysoganglioside $G_{M1}$, les produits d'hydrolyse partielle du ganglioside $G_{M1}$, et les mono- ou a-sialo gangliosides résultant du traitement acide des gangliosides ou de leurs dérivés.

4. Médicament selon l'une quelconque des revendications précédentes, caractérisé par le fait que le support est constitué de particules de charbon activé.

5. Médicament selon l'une quelconque des revendications précédentes, caractérisé par le fait que le dérivé de ganglioside est un produit de désacylation partielle de ganglioside.

6. Médicament selon la revendication 5, caractérisé par le fait que le produit de désacylation partielle est un dérivé du ganglioside $G_{M1}$.

7. Médicament selon l'une quelconque des revendications 1 à 3, caractérisé par le fait que le dérivé de ganglioside est le lysoganglioside $G_{M1}$.

8. Médicament selon l'une quelconque des revendications 5 à 7, caractérisé par le fait que ledit support est un support de cellulose modifiée ou non.

9. Médicament selon l'une quelconque des revendications précédentes, caractérisé par le fait qu'il contient de 1 à 20 micromoles environ de gangliosides ou de dérivés de gangliosides, par gramme de support.

10. Médicament selon la revendication 9, caractérisé par le fait qu'il contient environ 3 à 10 micromoles de gangliosides ou de dérivés de ganglioside par gramme de support.

11. Composition pharmaceutique, caractérisée par le fait qu'elle renferme comme principe actif un médicament tel que défini dans l'une quelconque des revendications précédentes.

12. Composition selon la revendication 11, caractérisée par le fait qu'elle contient en outre un excipient permettant de mettre ladite composition sous une forme galénique compatible avec l'administration par voie orale.

13. Composition selon l'une quelconque des revendications 11 et 12, caractérisée par le fait qu'elle contient de 0,1 à 20, et en particulier 0,1 à 10 micromoles environ de gangliosides ou de dérivés de gangliosides par gramme de composition.

14. Composition pharmaceutique selon l'une quelconque des revendications 11 à 13, caractérisée par le fait qu'elle se présente sous la forme de paquets ou sachets de poudre, de cachets ou gélules, de granulés, de comprimés, de suspensions buvables ou de capsules molles.

15. Composition pharmaceutique selon l'une quelconque des revendications 11 à 14, caractérisée par le fait qu'elle contient en outre au moins un autre produit actif choisi parmi les antiseptiques intestinaux, les antibiotiques et les antispasmodiques.

16. Composition selon l'une quelconque des revendications 11 à 15, caractérisée par le fait qu'elle contient en outre au moins une substance tensio-active.

**Claims**

1. Novel medicament, characterised in that it consists of a carrier onto which is fixed a ganglioside or a ganglioside derivative possessing affinity for toxins produced by bacteria responsible for diarrhoeas and especially for chorela toxin, the said carrier being compatible with oral administration.

2. Medicament according to Claim 1, characterised in that the carrier is chosen from the group consisting of activated charcoal, latex, modified or unmodified cellulose, porous inorganic carriers, such as silica, alumina and titanium oxide or their natural or synthetic derivatives such as glasses, silicates or kaolin.

3. Medicament according to any one of the preceding claims, characterised in that the ganglioside or the ganglioside derivative is chosen from the group consisting of ganglioside $G_{M1}$, lysoganglioside $G_{M1}$, the products of partial hydrolysis of ganglioside $G_{M1}$ and the monosialo-gangliosides or asialo-gangliosides resulting from the acid treatment of gangliosides or of their derivatives.

4. Medicament according to any one of the preceding claims, characterised in that the carrier consists of particles of activated charcoal.

5. Medicament according to any one of the preceding claims, characterised in that the ganglioside derivative is a product of the partial desacetylation of a ganglioside.

6. Medicament according to Claim 5, characterised in that the product of the partial desacetylation is a derivative of ganglioside $G_{M1}$.

7. Medicament according to any one of Claims 1 to 3, characterised in that the ganglioside derivative is lysoganglioside $G_{M1}$.

8. Medicament according to any one of Claims 5 to 7, characterised in that the said carrier is a modified or unmodified cellulose carrier.

9. Medicament according to any one of the preceding claims, characterised in that it contains approximately from 1 to 20 micromoles of gangliosides or of ganglioside derivatives per gram of carrier.

10. Medicament according to Claim 9, characterised in that it contains approximately from 3 to 10 micromoles of gangliosides or of ganglioside derivatives per gram of carrier.

11. Pharmaceutical composition, characterised in that it contains, as the active principle, a medicament as defined in any one of the preceding claims.

12. Composition according to Claim 11, characterised in that it moreover contains an excipient which allows the said composition to be converted to a galenical form compatible with oral administration.

13. Composition according to either of Claims 11 and 12, characterised in that it contains from 0.1 to 20, and in particular approximately from 0.1 to 10, micromoles of gangliosides or of ganglioside derivatives per gram of composition.

14. Pharmaceutical composition according to any one of Claims 11 to 13, characterised in that it is in the form packets or sachets of powder, of cachets or pills, of granules, of tablets, of potable suspensions or of soft capsules.

15. Pharmaceutical composition according to any one of Claims 11 to 14, characterised in that it moreover contains at least one other active product chosen from among intestinal antiseptics, antibiotics and antispasmodics.

16. Composition according to any one of Claims 11 to 15, characterised in that it moreover contains at least one surface-active substance.

**Ansprüche**

1. Neues Arzneimittel, dadurch gekennzeichnet, daß es aus einem Träger besteht, auf welchen ein Gangliosid oder Gangliosid-Derivat fixiert ist, das eine Affinität gegenüber Toxinen, welche durch Diarrhoe verursachende Bakterien gebildet werden, insbesondere gegenüber Choleratoxin besitzt, wobei dieser Träger bei oraler Verabreichung verträglich ist.

2. Arzneimittel nach Anspruch 1, dadurch gekennzeichnet, daß der Träger ausgewählt ist aus der Gruppe bestehend aus : Aktivkohle, Latex, modifizierte oder nicht modifizierte Cellulose, poröse mineralische Träger, wie Siliciumdioxid, Aluminiumoxid, Titanoxid oder deren natürliche oder synthetische Derivate, wie Gläser, Silikate oder Kaolin.

3. Arzneimittel nach den vorhergehenden Ansprüchen, dadurch gekennzeichnet, daß das Gangliosid oder Gangliosid-Derivat ausgewählt ist aus der Gruppe bestehend aus : Gangliosid $G_{M1}$, Lysogangliosid $G_{M1}$, den partiellen Hydrolysenprodukten von Gangliosid $G_{M1}$ und den Mono- oder A-Sialogangliosiden, die bei der Säurebehandlung der Ganglioside oder deren Derivate erhalten werden.

4. Arzneimittel nach den vorhergehenden Ansprüchen, dadurch gekennzeichnet, daß der Träger aus Aktivkohleteilchen besteht.

5. Arzneimittel nach den vorhergehenden Ansprüchen, dadurch gekennzeichnet, daß das Gangliosid-Derivat ein partielles Deacylierungsprodukt des Gangliosids ist.

6. Arzneimittel nach Anspruch 5, dadurch gekennzeichnet, daß das partielle Deacylierungsprodukt ein Derivat des Gangliosids $G_{M1}$ ist.

7. Arzneimittel nach den Ansprüchen 1 bis 3, dadurch gekennzeichnet, daß das Gangliosidderivat das Lysogangliosid $G_{M1}$ ist.

8. Arzneimittel nach den Ansprüchen 5 bis 7, dadurch gekennzeichnet, daß der Träger ein modifizierter oder nicht modifizierter Celluloseträger ist.

9. Arzneimittel nach den vorhergehenden Ansprüchen, dadurch gekennzeichnet, daß es etwa 1 bis 20 Mikromol Ganglioside oder Gangliosidderivate je Gramm Träger enthält.

10. Arzneimittel nach Anspruch 9, dadurch gekennzeichnet, daß es etwa 3-10 Mikromol Ganglioside oder Gangliosidderivate je Gramm Träger enthält.

11. Pharmazeutische Zubereitung, dadurch gekennzeichnet, daß sie als aktiven Wirkstoff ein Arzneimittel nach den vorhergehenden Ansprüchen enthält.

12. Zubereitung nach Anspruch 11, dadurch gekennzeichnet, daß sie weiterhin einen Exzipienten enthält, der es gestattet, die Zubereitung in einer galenischen Form einzubringen, die mit einer oralen Verabreichung verträglich ist.

13. Zubereitung nach den Ansprüchen 11 und 12, dadurch gekennzeichnet, daß sie etwa 0,1 bis 20, insbesondere 0,1 bis 10 Mikromol Gangliosid oder Gangliosidderivat je Gramm Zubereitung enthält.

14. Pharmazeutische Zubereitung nach den Ansprüchen 11-13, dadurch gekennzeichnet, daß sie in der Form von Pulverpaketen oder -säcken, Tabletten, Kapseln, Körner, Kompretten, trinkbaren Suspensionen oder Weichkapseln vorliegt.

15. Pharmazeutische Zubereitung nach den Ansprüchen 11-14, dadurch gekennzeichnet, daß sie weiterhin mindestens einen weiteren Wirkstoff enthalten, ausgewählt aus den Darmantiseptika, Antibiotika und Antispasmolytika.

16. Zubereitung nach den Ansprüchen 11-15, dadurch gekennzeichnet, daß sie weiterhin mindestens eine oberflächenaktive Substanz enthalten.